# EUROPEAN PATENT APPLICATION

(11) **EP 2 412 696 A1**
(43) Date of publication of application: **01.02.2012**
(21) Application number: 09842264.5
(22) Date of filing: 27.03.2009
(51) Int. Cl.: C07C 45/74, C07C 45/62, C07C 45/82, C07C 49/385, C07C 49/587, B01J 21/10, B01J 23/02, B01J 23/06, C07B 53/00, C07B 61/00

(54) **PROCESS FOR PRODUCTION OF 3-METHYL-CYCLOPENTADECENONE, PROCESS FOR PRODUCTION OF R/S-MUSCONE, AND PROCESS FOR PRODUCTION OF OPTICALLY ACTIVE MUSCONE**

(71) Applicant: Doya, Masaharu, Niigata 950-0011 (JP)
(72) Inventor: Doya, Masaharu, Niigata 950-0011 (JP)
(74) Representative: Jolly, Mark Edward
(86) International application number: PCT/JP2009/056302
(87) International publication number: WO 2010/109650

(57) **Abstract**

By intramolecular condensation reaction of 2,15-hexadecanedione in a gaseous phase with a compound of a Group II element of the Periodic Table as a catalyst, 3-methyl-cyclopentadecenones is generated. Magnesium oxide, calcium oxide, or zinc oxide is desirable as the catalyst for the intramolecular condensation reaction. (R)- and (S)-muscone is generated by subjecting 3-methyl-cyclopentadecenones obtained as above to hydrogenation by using a catalyst. Palladium catalyst is desirable as the hydrogenation catalyst. Optically active muscone is generated by separating 3-methyl-cyclopentadecenones into respective components thereof by means of precision distillation and subsequently subjecting the separated 3-methyl-cyclopentadecenones to asymmetric hydrogenation by using an optically active ruthenium complex catalyst. The production methods described above enable easy and economical production of 3-methyl-cyclopentadecenones, (R)-and (S)-muscone, and optically active muscone.

## Description

### TECHNICAL FIELD

The present invention relates to a method for producing 3-methyl-cyclopentadecenones, which are synthetic intermediates for muscone, a compound useful as a perfuming ingredient. The present invention further relates to a method for producing (R)- and (S)-muscone and a method for producing optically active muscone.

### BACKGROUND ART

Conventionally known examples of methods for producing 3-methyl-cyclopentadecenones, which are synthetic intermediates for producing muscone, include a method that involves intramolecular cyclization of 2,15-hexadecanedione by using an organozinc compound in the presence of an inert solvent (e. g. see Patent Document 1). Another known example of a method involves intramolecular cyclization of 2,15-hexadecanedione in a gaseous phase at a temperature between 300 and 400°C using TiO₂, CeO₂, or ThO₂ as a catalyst in the presence of water of 5 to 15 W/W% to the quantity of the catalyst (e. g. see Patent Document 2). Yet another known example of a method relates to a method for obtaining (E)-3-methyl-2-cyclopentadecenone by subjecting 3-hydroxy-3-methylcyclopentadecanone to a dehydration reaction using an alkoxytitanium compound (e. g. see Patent Document 3).

Conventionally known examples of methods for producing optically active muscone from 3-methyl-2-cyclopentadecenone include a method that involves separating and purifying (E)-3-methyl-2-cyclopentadecenone and (Z)-3-methyl-2-cyclopentadecenone from a mixture containing (E)- and (Z)-3-methyl-2-cyclopentadecenones by means of column chromatography, and subjecting the (E)-3-methyl-2-cyclopentadecenone and the (Z)-3-methyl-2-cyclopentadecenone obtained as above to asymmetric hydrogenation by using a ruthenium-optically active phosphine complex (e. g. see Patent Document 4).
Patent Document 1: Japanese Laid-open Patent Publication No. 59-157047 (pp 2 and 3)
Patent Document 2: Japanese Laid-open Patent Publication No. 3-81242 (pp 3 to 6)
Patent Document 3: Japanese Laid-open Patent Publication No. 2002-69026 (pp 3 to 5)
Patent Document 4: Japanese Laid-open Patent Publication No. 6-192161 (pp 3 and 4)

### DISCLOSURE OF THE INVENTION

### Problems to be Solved by The Invention

However, all the conventional methods described above for producing 3-methyl-cyclopentadecenones present the problem of being not economical, because of high production costs due to such reasons as requiring a large quantity of a generally expensive catalyst, a special catalyst, or a high dilution system, or being prone to a low yield.

Furthermore, according to the methods for producing muscone described above, (R)- and (S)-muscone can easily be produced by using a hydrogenation catalyst. However, production of optically active muscone requires a highly purified geometrically isomeric 3-methyl-2-cyclopentadecenone, which is difficult to produce, resulting in the possibility of an increase in the production cost. Therefore, the methods present the problem of being uneconomical.

For example, the method disclosed in Patent Document 1 involves liquid phase reaction and therefore requires a high dilution system (in the case of the Example, the concentration of 2,15-hexadecanedione, which is the raw material, is approximately 0.2wt/vol%) in order to suppress intermolecular condensation, and also necessitates use of a great quantity of ethylzinc iodide, a catalyst that is generally expensive. As these requirements result in high production costs, the method disclosed in Patent Document 1 is not economical.

The method disclosed in Patent Document 2 is a method for obtaining (R)- and (S)-muscone by using TiO₂, CeO₂, or ThO₂ as a catalyst to allow reaction to take place in a gaseous phase in order to suppress intermolecular condensation, and performing hydrogenation of the resulting 3-methyl-cyclopentadecenones by a method known to those skilled in the art. However, this method, too, presents a problem of not being economical, because it requires a special treatment, such as doping the catalyst with an oxide of an alkali metal or of an alkaline earth metal in order to increase the selectivity of the 3-methyl-cyclopentadecenones.

The method disclosed in Patent Document 3 is a method for producing (E)-3-methyl-2-cyclopentadecenone via 3-hydroxy-3-methylcyclopentadecanone by using 2,15-hexadecanedione as a starting raw material. However, the method not only requires a high dilution system in order to suppress intermolecular linking at the stage of synthesizing the 3-hydroxy-3-methylcyclopentadecanone but also is prone to a low yield, i.e. 38%, in spite of having to use a large quantity of auxiliary materials, such as tributylamine and titanium tetrachloride. Furthermore, in addition to requiring use of a great quantity of orthotitanic acid ester as an auxiliary material at the stage of producing (E)-3-methyl-2-cyclopentadecenone, the method also requires such costly treatments as purification using column chromatography due to generation of a small quantity of (Z)-isomer as a by-product. Therefore, this method, too, presents a problem of not being economical.

The method disclosed in Patent Document 4 is a method involving asymmetric hydrogenation of geometrically isomeric (E)-3-methyl-2-cyclopentadecenone by using a ruthenium-optically active phosphine complex as a catalyst, and is excellent as a method for producing optically active muscone. However, as is true in the method disclosed in Patent Document 3, the method disclosed in Patent Document 4 is not economical, because it is difficult to produce at low cost geometrically isomeric 3-methyl-2-cyclopentadecenone, which is the raw material.

In order to solve the above problems, an object of the invention is to provide easy and economical methods for producing 3-methyl-cyclopentadecenones, (R)- and (S)-muscone, and optically active muscone.

### Means to Solve The Problems

A method for producing 3-methyl-cyclopentadecenones according to claim 1 of the present invention involves subjecting 2,15-hexadecanedione in a gaseous phase to intramolecular condensation reaction in the presence of a compound that includes a Group II element of the Periodic Table and is used as a catalyst.

According to claim 2 of the present invention, the compound that includes a Group II element of the Periodic Table and is used as the catalyst in the method for producing 3-methyl-cyclopentadecenones according to claim 1 of the present invention is selected from the group consisting of magnesium oxide, calcium oxide, and zinc oxide.

A method for producing (R)- and (S)-muscone according to claim 3 of the present invention is characterized by subjecting 3-methyl-cyclopentadecenones produced by the method for producing 3-methyl-cyclopentadecenones according claim 1 or 2 of the present invention to hydrogenation using a catalyst.

A method for producing 3-methyl-cyclopentadecenones according to claim 4 of the present invention is **characterized in that** 3-methyl-cyclopentadecenones that contain at least (E)-3-methyl-2-cyclopentadecenone and (Z)-3-methyl-2-cyclopentadecenone are separated into respective components by means of precision distillation.

A method for producing optically active muscone according to claim 5 of the present invention is characterized by subjecting 3-methyl-cyclopentadecenones produced by the method for producing 3-methyl-cyclopentadecenones according to claim 4 of the present invention to asymmetric hydrogenation by using an optically active ruthenium complex catalyst.

### Effects of The Invention

According to claim 1 of the present invention, a compound of a Group II element of the Periodic Table is used, and intramolecular condensation reaction is allowed to take place in a gaseous phase. Therefore, 3-methyl-cyclopentadecenones can be produced easily and economically.

According to claim 2 of the present invention, 3-methyl-cyclopentadecenones can be produced economically, because the compound of a Group II element of the Periodic Table is selected from the group consisting of magnesium oxide, calcium oxide, and zinc oxide.

According to claim 3 of the present invention, (R)- and (S)-muscone can be produced easily and economically, because the method merely requires subjecting 3-methyl-cyclopentadecenones produced by the method for producing 3-methyl-cyclopentadecenones according to claim 1 or 2 of the present invention to hydrogenation using a catalyst.

According to claim 4 of the present invention, 3-methyl-cyclopentadecenones can be produced easily and economically by separation into respective components thereof by means of precision distillation.

According to claim 5 of the present invention, optically active muscone can be produced easily and economically by subjecting 3-methyl-cyclopentadecenones produced by the method for producing 3-methyl-cyclopentadecenones according to claim 4 of the present invention to asymmetric hydrogenation by using an optically active ruthenium complex catalyst.

### BEST MODE FOR CARRYING OUT THE INVENTION

Next, methods for respectively producing 3-methyl-cyclopentadecenones, (R)- and (S)-muscone, and optically active muscone according to an embodiment of the present invention are explained in detail hereunder.

First of all, the method for producing 3-methyl-cyclopentadecenones is explained.

3-methyl-cyclopentadecenones can be obtained by introducing a raw material, i.e. 2,15-hexadecanedione, in a gaseous phase into a reaction tube filled with a catalyst, and subjecting the 2,15-hexadecanedione to intramolecular condensation reaction.

The catalyst used is a compound of a Group II element of the Periodic Table. Magnesium oxide, calcium oxide, and zinc oxide are particularly desirable examples, of which any compound or a mixture of compounds may be used alone or in combination with a forming agent that is inert to the reaction. Furthermore, although the catalyst is usually in the form of pellets or tablets, there are no particular limitations as to the shape of the catalyst.

In intramolecular condensation reaction, a solvent or inert gas is used in order to suppress intermolecular condensation, which is a side reaction. The raw material, i.e. 2,15-hexadecanedione, is dissolved in a solvent and then gasified in a vaporization tube or an evaporator in the presence of inert gas that serves as a carrier gas, and, thereafter, introduced into the reaction tube filled with the catalyst.

A hydrocarbon is normally used as the solvent. Although aliphatic hydrocarbons with 6 to 12 carbon atoms are particularly desirable, there are no particular limitations, provided that the solvent is inert to the reaction. To be more specific, examples of compounds that can be used as a solvent include toluene, xylene, decalin, and decane. As the solvent used in excessive quantity is not economical while that in insufficient quantity is not capable of suppressing the side reaction, the desirable quantity of the solvent used is usually in the range of 10 to 100 times the weight of the 2,15-hexadecanedione that is the raw material. However, the quantity of the solvent is not limited to the abovementioned range and may be set as desired.

Although carbon dioxide or nitrogen gas is typically used as the inert gas, there are no particular limitations as to what can be used as the inert gas, provided that the gas is inert to the reaction. As the inert gas used in excessive quantity is not economical while that in insufficient quantity is not capable of suppressing the side reaction, 1 to 20 L of the inert gas is usually used for every gram of the raw material 2,15-hexadecanedione.

Although the temperature in the section where vaporization takes place is normally in the range of 200 to 350°C, the temperature is not limited to this range, provided that the temperature is sufficient to ensure vaporization of all the raw material 2,15-hexadecanedione.

A reaction temperature that is too low inhibits the progress of the reaction, while an excessively high temperature causes decomposition reaction. Therefore, the temperature is controlled within the range of 300 to 400°C, preferably 350 to 380°C.

Should the raw material 2,15-hexadecanedione be introduced into a catalyst layer too fast, a reduction in the conversion rate of the 2,15-hexadecanedione occurs. On the other hand, should 2,15-hexadecanedione be introduced too slowly, the side reaction increases, resulting in reduction in the selectivity of the 3-methyl-cyclopentadecenones as well as reduction in catalytic activity. Therefore, LHSV of the raw material 2,15-hexadecanedione is limited within the range of 0.002 to 0.05.

During the reaction, an excessively high conversion rate of the raw material 2,15-hexadecanedione reduces the selectivity of the 3-methyl-cyclopentadecenones, which is the target substance. On the other hand, an excessively low conversion rate is uneconomical, although the selectivity of the 3-methyl-cyclopentadecenones, which is the target substance, improves. Therefore, the conversion rate of the raw material 2,15-hexadecanedione should desirably be limited within the range of 40 to 80%.

The catalytic activity gradually decreases with the elapse of reaction time. However, the usable catalyst life until reactivation of the catalyst can be increased by gradually increasing the reaction temperature.

At the moment when the selectivity of the 3-methyl-cyclopentadecenones starts to decrease instead of increase in spite of having increased the temperature to 380°C, the supply of the raw material is halted, and reactivation of the catalyst is performed.

The catalyst is reactivated by introducing air or oxygen into the catalyst layer and removing by incineration high-boiling-point by-products that have accumulated in the catalyst layer. There are no limitations as to the introduction rate of air. Furthermore, the reactivation is performed at a temperature of 400°C or higher, preferably in the range of 450 to 500°C.

The reaction product can be obtained in a liquid state by collecting the product at a temperature of 30 to 60°C. The reaction product primarily consists of the solvent used, 3-methyl-cyclopentadecenones, and unreacted 2,15-hexadecanedione.

By further cooling the reaction product liquid that has been obtained, the majority of the unreacted 2,15-hexadecanedione can be separated by crystallization. The unreacted 2,15-hexadecanedione that has been recovered can be circulated for reuse.

After the separation of the unreacted 2,15-hexadecanedione, the liquid containing 3-methyl-cyclopentadecenones can be used in hydrogenation for producing (R)- and (S)-muscone.

As described above, 3-methyl-cyclopentadecenones can be produced by intramolecular condensation reaction of 2,15-hexadecanedione in a gaseous phase with a compound of a Group II element of the Periodic Table as a catalyst. As this method neither requires use of a special solvent nor suppression of intermolecular condensation reaction by a great degree of dilution or other means, the method enables easy and economical production of 3-methyl-cyclopentadecenones.

Furthermore, by using magnesium oxide, calcium oxide, or zinc oxide as the compound of a Group II element of the Periodic Table, 3-methyl-cyclopentadecenones can be produced easily and economically, because the abovementioned compounds are generally easy to acquire.

Furthermore, recovering the unreacted 2,15-hexadecanedione for reuse through circulation enables efficient use of 2,15-hexadecanedione, resulting in more economical production of 3-methyl-cyclopentadecenones.

Furthermore, the 2,15-hexadecanedione used in the intramolecular condensation reaction described above may be an aliphatic diketone produced by a method for producing aliphatic diketone that involves reaction between aliphatic diiodide and ketones in the presence of an inorganic alkaline compound.

Next, the method for producing (R)- and (S)-muscone is explained.

As described above, (R)- and (S)-muscone can easily be obtained by separating the majority of the unreacted 2,15-hexadecanedione from the reaction product liquid that has resulted from intramolecular condensation reaction of 2,15-hexadecanedione, and hydrogenating the resulting liquid that contains 3-methyl-cyclopentadecenones using a catalyst. Hydrogenation by the catalyst is performed either directly after the separation of the unreacted 2,15-hexadecanedione, in other words by using the liquid containing 3-methyl-cyclopentadecenones as is, or after removing impurities therefrom by means of distillation.

Hydrogenation may be performed by a variety of methods, such as a method that involves adding a hydrogenation catalyst to the liquid that contains 3-methyl-cyclopentadecenones and subsequently bubbling hydrogen, a method that involves pressurizing hydrogen at 0 to 100 kg/cm² using an autoclave, or a flowing method involving co-current of the raw material and hydrogen into a catalyst that fills a reaction tube.

Examples of the catalyst that can be used include nickel catalyst, cobalt catalyst, copper catalyst, palladium catalyst, platinum catalyst, ruthenium catalyst, and rhodium catalyst, of which palladium catalyst is particularly desirable.

The quantity of the catalyst used may be set appropriately based on the kind and activity of the catalyst, the reaction temperature, or the like. Normally, however, the quantity is in the range of 0.001 to 0.1 of the weight of the 3-methyl-cyclopentadecenones.

It is desirable to perform hydrogenation by using a solvent. Although there are no particular limitations as to the solvent, provided that it is inert to the hydrogenation, it is desirable from the perspective of efficiency to use the hydrocarbon that has been used for the intramolecular condensation reaction of 2,15-hexadecanedione described above.

As the solvent used in an excessive quantity is not economical, the quantity of the solvent is limited so as to make the concentration of the 3-methyl-cyclopentadecenones not lower than 1 W/W%.

Although the reaction temperature should vary depending on the kinds of catalyst and solvent, it is normally controlled within the range of room temperature to 100°C.

(R)- and (S)-muscone can be obtained by purifying, by distillation or column chromatography, the reaction product obtained through the hydrogenation described above.

Furthermore, even if a small quantity of 2,15-hexadecanedione is contained in the raw material liquid for the hydrogenation, 2,15-hexadecanedione is not only inert to the hydrogenation but also is recovered as the bottom liquid at the time of purification by distillation. The 2,15-hexadecanedione that has been recovered can be circulated to the process in which separation of the unreacted material is carried out by crystallization so that the recovered 2,15-hexadecanedione can be used again for intramolecular condensation reaction.

As described above, 3-methyl-cyclopentadecenones produced by the method for producing 3-methyl-cyclopentadecenones described above are hydrogenated by using a catalyst so as to produce (R)- and (S)-muscone. As this method for producing (R)- and (S)-muscone requires neither a special catalyst nor special treatment, the method enables easy and economical production of (R)- and (S)-muscone.

Next, the method for separating various components from a liquid containing 3-methyl-cyclopentadecenones by means of precision distillation is explained.

Principal constituents of the liquid containing 3-methyl-cyclopentadecenones from which the majority of the unreacted 2,15-hexadecanedione has been separated are the solvent used for the reaction, 3-methyl-cyclopentadecenones, and a small quantity of unreacted 2,15-hexadecanedione. Precision distillation is performed after the solvent is removed by distillation.

Examples of the 3-methyl-cyclopentadecenones that can be obtained by intramolecular condensation reaction of 2,15-hexadecanedione include (E)-3-methyl-2-cyclopentadecenone, (Z)-3-methyl-2-cyclopentadecenone, (E)- and (Z)-3-methyl-3-cyclopentadecenone, and 3-methylene-cyclopentadecanone, out of which at least (E)-3-methyl-2-cyclopentadecenone and (Z)-3-methyl-2-cyclopentadecenone are always contained.

It is sufficient that a distillation column used for the precision distillation has 30 or more theoretical plates. Examples of columns that can be used include a packed column, plate column, and a spinning band column.

As insufficient pressure reduction during distillation increases the distillation temperature and consequently causes decomposition of feed composition, a high degree of vacuum is desirable. It is desirable to be set in the range of 0.5 to 50 mmHg.

The distillation temperature is determined by the raw material composition and the degree of vacuum. It is desirable that the vapor temperature at the column top be in the range of 100 to 200°C.

As the reflux ratio is affected by the composition of the feed liquid, a sweeping generalization cannot be made about the reflux ratio. However, in order to obtain high-purity products at high separation yield, the reflux ratio has to be not less than 30.

The components of the 3-methyl-cyclopentadecenones obtained by precision distillation can be used as raw materials for producing optically active muscone.

Furthermore, 3-methyl-cyclopentadecenones of which the components are separated by means of precision distillation as described above are not limited to those obtained by intramolecular condensation reaction of 2,15-hexadecanedione in a gaseous phase by using a catalyst and subsequent removal of the majority of the unreacted 2,15-hexadecanedione. It is sufficient that the 3-methyl-cyclopentadecenones contain at least (E)-3-methyl-2-cyclopentadecenone and (Z)-3-methyl-2-cyclopentadecenone.

As described above, 3-methyl-cyclopentadecenones that contain at least (E)-3-methyl-2-cyclopentadecenone and (Z)-3-methyl-2-cyclopentadecenone are separated into each component by means of precision distillation, without requiring column chromatography, which generally results in high production costs. Therefore, the method described above enables easy and economical production of 3-methyl-cyclopentadecenones.

Next, the method for producing optically active muscone is explained.

Optically active muscone can be obtained by subjecting 3-methyl-cyclopentadecenones that have been obtained by precision distillation as described above to asymmetric hydrogenation by using an optically active ruthenium complex catalyst.

Asymmetric hydrogenation may be performed by a variety of methods, such as a method that involves bubbling hydrogen into a liquid containing a component of 3-methyl-cyclopentadecenones and an optically active ruthenium complex catalyst, a method that involves pressurizing hydrogen at 0 to 100 kg/cm² using an autoclave, or a flowing method involving co-current of the raw material and hydrogen into the catalyst that fills a reaction tube.

With regard to examples of the optically active ruthenium complex catalyst, it is desirable, as in the case of the method for producing optically active muscone disclosed in Japanese Laid-open Patent Publication No. 6-192161, to use a ruthenium-optically active phosphine complex, examples of which include Ru₂Cl₄(BINAP)₂(NEt₃), Ru₂Cl₄(Tol-BINAP)₂(NEt₃), Ru₂Cl₄(t-Bu-BINAP)₂(NEt₃). Ru (BINAP) (OAc)₂, Ru (Tol-BINAP) (OAc)₂, or Ru (t-Bu-BINAP)(OAc)₂. The term "BINAP," "Tol-BINAP," and "t-Bu-BINAP" mentioned above represent 2,2'-bis(diphenylphosphino)-1,1'binaphthyl, 2,2'-bis(ditolylphosphino)-1,1'binaphthyl, and 2,2'-bis(dip-tert-butylphenylphosphino)-1,1'binaphthyl, respectively.

The ruthenium-optically active phosphine complex mentioned above is in R configuration or S configuration, and either configuration can be appropriately selected to produce optically active muscone.

The quantity of the catalyst used may be set appropriately based on the kind and activity of the catalyst, the reaction temperature, or the like. However, it is desirable that the quantity be in the range of 0.0001 to 0.05 of the weight of the 3-methyl-cyclopentadecenones.

It is desirable to perform asymmetric hydrogenation by using a solvent. Although there are no particular limitations as to the solvent, provided that it is inert to the asymmetric hydrogenation, examples of such a solvent include alcohols, hydrocarbons, and halogenated hydrocarbons. As the solvent used in an excessive quantity is not economical, the quantity of the solvent is desirably limited so as to make the concentration of the 3-methyl-cyclopentadecenones not lower than 1 W/W%.

Although the asymmetric hydrogenation temperature should be set depending on the kinds of catalyst and solvent, it is desirably set within the range of room temperature to 100°C.

Optically active muscone can be obtained by purifying, by distillation or column chromatography, the reaction product obtained through the hydrogenation described above.

As described above, optically active muscone can be easily and economically produced by subjecting 3-methyl-cyclopentadecenones that have been produced by the method for producing 3-methyl-cyclopentadecenones by means of precision distillation to asymmetric hydrogenation using an optically active ruthenium complex catalyst.

Furthermore, any one of the methods described above, i.e. the method for producing 3-methyl-cyclopentadecenones, the method for producing (R)- and (S)-muscone, the method for separating each component from a liquid containing 3-methyl-cyclopentadecenones by means of precision distillation, and the method for producing optically active muscone, may be applicable to a batch process or a continuous process whenever it is appropriate.

### Examples

Next, actual examples according to the present invention are explained hereunder.

First of all, production of 2,15-hexadecanedione to be used for producing 3-methyl-cyclopentadecenones is explained as a reference example.

Measured into a 2-liter four-necked flask provided with a stirring device, a thermometer, and a reflux condenser were 197g (0.5 mol) of 1,10-diiododecane, 520g (4 mol) of ethyl acetoacetate, 1 L of ethanol, and 89.8g (0.65 mol) of potassium carbonate, with reaction subsequently being allowed to take place for four hours under total reflux.

After the reaction was completed, the ethanol, which was used as the solvent, was removed by distillation, and the remaining liquid was cooled to room temperature and subjected to liquid separation by adding 700 ml of 5% sulfuric acid. After the surplus of ethyl acetoacetate was removed by reduced pressure distillation of the organic layer at the upper layer. As a result, 237g of an oily substance containing diethyl-2,13-bisacetyl-1,14-tetradecandioate was obtained.

All the oil substance obtained as described above and 800g (2 mol) of 10% aqueous solution of sodium hydroxide were put into a 2-liter three-necked flask provided with a stirring device, a thermometer, and a reflux condenser, and stirred for five hours at room temperature. Thereafter, 206g (1.05 mol) of 50% sulfuric acid was added, and decarboxylation was allowed to take place for three hours under total reflux.

After the decarboxylation reaction was completed, the temperature was reduced to room temperature. Then, the solid substance was filtered out, washed with water, and dried so that 129.6g of faintly yellow crystals were obtained. The result of gas chromatography analysis of the composition of the obtained crystals indicated that the concentrations of the 1,10-diiododecane and 2,15-hexadecanedione were respectively 0 W/W% and 91.3 W/W%. Therefore, the conversion rate of the 1,10-diiododecane was 100%, while the selectivity of the 2,15-hexadecanedione was 93.2%. In other words, the yield of 2,15-hexadecanedione with respect to the feed 1,10-diiododecane was 93.2%.

All the crude 2,15-hexadecanedione that had been obtained was purified by recrystallization using 95% ethanol. As a result, 110g of purified 2,15-hexadecanedione with a purity of not less than 99.5% was obtained.

Then, the 2,15-hexadecanedione that had been produced as described above was subjected in a gaseous phase to intramolecular condensation reaction in the presence of a catalyst to obtain 3-methyl-cyclopentadecenones.

### [Example 1]

An upper part of a column with a diameter of 22 mm and a length of 40 cm was filled with 35 ml of ceramic Raschig rings having 3 to 4 mm diameter, and a lower part of the column was filled with 50 ml of 3 to 5 mm diameter pellets of zinc oxide, which is a compound of a Group II element of the Periodic Table and served as the catalyst. The column was then heated so that the temperatures of the Raschig ring layer and the catalyst layer were respectively 315°C and 360°C. In the presence of 5 L/hr of nitrogen, which is inert gas serving as the carrier gas, a toluene-decalin solution with a volume ratio of 1:3 in which 5 w/w% of 2,15-hexadecanedione was dissolved was introduced into the heated column at a rate of 25 g/hr and subjected to intramolecular condensation reaction. The reaction product resulting from the intramolecular condensation reaction was cooled to a temperature in the range of 30 to 50°C and collected.

A continuous reaction was allowed to take place for three hours. Upon the elapse of the three hours, the reaction product liquid was analyzed by gas chromatography. The result of the analysis indicated that the conversion rate of the 2,15-hexadecanedione and the selectivity of the 3-methyl-cyclopentadecenones were 65% and 86%, respectively. Therefore, the yield of 3-methyl-cyclopentadecenones with respect to the feed 2,15-hexadecanedione was 56%.

### [Example 2 and Example 3]

Reactions were allowed to take place in the same manner as in Example 1 described above except that the catalysts used were respectively calcium oxide and magnesium oxide, both of which are compounds of Group II elements of the Periodic Table. The results are shown in Table 1.

**[Table 1]**

| Example | Kind of Catalyst | 2,15-hexadecanedione | 3-methyl-cyclopentadecenones | |
|---|---|---|---|---|
| | | conversion rate (%) | selectivity (%) | yield (%) |
| 2 | calcium oxide | 60 | 38 | 23 |
| 3 | magnesium oxide | 72 | 46 | 33 |

### [Comparative Example 1 to Comparative Example 10]

Reactions were allowed to take place in the same manner as in Example 1 described above except that the catalysts used were various compounds of elements that do no belong to Group II of the Periodic Table. The results are shown in Table 2.

**[Table 2]**

| Comparative Example | Kind of Catalyst | 2,15-hexadecanedione | 3-methyl-cyclopentadecenones | |
|---|---|---|---|---|
| | | conversion rate (%) | selectivity (%) | yield (%) |
| 1 | titanium (IV) oxide | 79 | 32 | 24 |
| 2 | zirconium (IV) oxide | 99 | 0 | 0 |
| 3 | manganese (II) oxide | 10 | 18 | 2 |
| 4 | iron (III) oxide | 4 | 0 | 0 |
| 5 | nickel oxide | 15 | 36 | 5 |
| 6 | lead oxide | 14 | 0 | 0 |
| 7 | graphite | 0 | - | - |
| 8 | molecular sieves (13X) | 100 | 0 | 0 |
| 9 | γ-alumina | 91 | 4 | 4 |
| 10 | ceramic Raschig rings | 0 | - | - |

### [Example 4]

A Raschig ring-filled tube, which is a tube having a diameter of 22 mm and a length of 30 cm and filled with 50 ml of ceramic Raschig rings having 3 to 4 mm diameter, is positioned above a catalyst-filled tube having a diameter of 22 mm and a length of 40 cm. The catalyst-filled tube was filled with 80 ml of 3 to 5 mm diameter pellets of zinc oxide, which is a compound of a Group II element of the Periodic Table and served as the catalyst. The tubes were then heated so that the temperatures of the Raschig ring-filled tube and the catalyst-filled tube were respectively 320°C and 360°C. In the presence of 5 L/hr of nitrogen serving as a carrier gas, an n-decane solution in which 5 w/w% of 2,15-hexadecanedione was dissolved was introduced into the Raschig ring-filled tube at a rate of 25 g/hr and subjected to intramolecular condensation reaction. The reaction product resulting from the intramolecular condensation reaction was cooled to a temperature in the range of 30 to 50°C and collected.

A continuous reaction was allowed to take place for ten hours. Upon the elapse of the ten hours, the reaction product liquid was analyzed by gas chromatography. The result of the analysis indicated that the conversion rate of the 2,15-hexadecanedione and the selectivity of the 3-methyl-cyclopentadecenones were 59% and 84%, respectively.

An inspection of the Raschig ring-filled tube and the catalyst-filled tube after the reaction found that a tar-like substance was attached to the Raschig ring layer and that the upper part of the catalyst layer had changed color from white to gray.

Furthermore, after heating the Raschig ring-filled tube and the catalyst-filled tube to a temperature of 450 to 500°C, and air was introduced at a rate of 0.5 L/min to incinerate the tar-like substance and reactivate the catalyst, the intramolecular condensation reaction described above was allowed to take place again. Upon the elapse of ten hours, the reaction product liquid was analyzed by gas chromatography. The result of the analysis indicated that the conversion rate of the 2,15-hexadecanedione and the selectivity of the 3-methyl-cyclopentadecenones were 61% and 82%, respectively.

Reactions described above, which involved reactivation of the catalyst, were repeated until a total of five reactions were allowed to take place. There was no recognizable decrease in the activity of the catalyst. Upon the elapse of ten hours of the fifth reaction, the reaction product liquid was analyzed by gas chromatography. The result of the analysis indicated that the conversion rate of the 2,15-hexadecanedione and the selectivity of the 3-methyl-cyclopentadecenones were 61% and 86%, respectively.

### [Example 5]

The reaction product liquid, amounting to 1,200 g, that resulted from Example 4 was collected and cooled to 20°C. By filtration separation of precipitated crystals, 1,158g of liquid containing 2.5 w/w% of 3-methyl-cyclopentadecenones and 0.1 w/w% of unreacted 2,15-hexadecanedione was obtained. The crystals of unreacted 2,15-hexadecanedione that had been recovered weighed 20.4g after drying and had a purity of 95 w/w%.

After the removal of the unreacted 2,15-hexadecanedione, 50g of the resulting liquid containing 3-methyl-cyclopentadecenones was collected, and 0.1g of 5 w/w% palladium on carbon was added as a catalyst to the liquid, which was then subjected to agitation for 6 hours at 25°C under pressurized hydrogen of 50 kg/cm², thereby performing hydrogenation.

The result of gas chromatography analysis of the composition of the liquid that was obtained after the reaction was completed indicated that (R)- and (S)-muscone, unreacted 3-methyl-cyclopentadecenones, and 2,15-hexadecanedione respectively amounted to 2.5 W/W%, not more than 0.1 W/W%, and 0.1 W/W%.

### [Example 6]

After the removal of the unreacted 2,15-hexadecanedione obtained in Example 5, 1,100g of the liquid containing 3-methyl-cyclopentadecenones was concentrated and subjected to precision distillation in a vacuum in the range of 10 to 2 mmHG and at a reflux ratio in the range of 10 to 100 by using a spinning band-type fractionating distillation apparatus (with 80 theoretical plates) made by TOKASEIKI Co. Ltd. After removing the initial fraction, the fraction in the amount of 10.3g was obtained in a vacuum at 2 mmHg and at a temperature in the range of 140.5 to 142°C. The result of gas chromatography analysis of the composition of the fraction indicated that the content of (Z)-3-methyl-2-cyclopentadecenone was 95.3 W/W%.

### [Example 7]

Put in a 100 ml pressure vessel filled with nitrogen were 2g of (Z)-3-methyl-2-cyclopentadecenone that was a fraction obtained in Example 6 described above, 10 mg of Ru₂Cl₄[(R)Tol-BINAP]₂(NEt₃) serving as the optically active ruthenium complex catalyst, and 15 ml of methanol serving as a solvent. The mixture was then subjected to agitation for 24 hours at 25°C under pressurized hydrogen of 50 kg/cm², thereby performing asymmetric hydrogenation.

After the reaction was completed, the methanol, which was used as the solvent, was removed by distillation. Thereafter, 1.9g of (R)-muscone, which is optically active muscone, was obtained by purifying the resulting crude reaction product by means of silica gel column chromatography. A liquid chromatography analysis of this (R)-muscone indicated that the ratio of (R)- and (S)-isomers was 93:7.

### INDUSTRIAL APPLICABILITY

The present invention is applicable to producing (R)-and (S)-muscone or optically active muscone, both of which are useful as perfuming ingredients, as well as producing 3-methyl-cyclopentadecenones, which are intermediates for muscone.

## Claims

1. A method for producing 3-methyl-cyclopentadecenones comprising:
subjecting 2,15-hexadecanedione in a gaseous phase to intramolecular condensation reaction in the presence of a compound of a Group II element of the Periodic Table, said compound of a Group II element being used as a catalyst.

2. A method for producing 3-methyl-cyclopentadecenones as claimed in claim 1, wherein:
the compound of a Group II element used as the catalyst is selected from the group consisting of magnesium oxide, calcium oxide, and zinc oxide.

3. A method for producing (R)- and (S)-muscone, wherein:
3-methyl-cyclopentadecenones produced by the method for producing 3-methyl-cyclopentadecenones as claimed in claim 1 or claim 2 are subjected to hydrogenation using a catalyst.

4. A method for producing 3-methyl-cyclopentadecenones, wherein:
3-methyl-cyclopentadecenones that contain at least (E)-3-methyl-2-cyclopentadecenone and (Z)-3-methyl-2-cyclopentadecenone are separated into respective components by means of precision distillation.

5. A method for producing optically active muscone, wherein:
3-methyl-cyclopentadecenones produced by the method for producing 3-methyl-cyclopentadecenones as claimed in claim 4 are subjected to asymmetric hydrogenation using an optically active ruthenium complex catalyst.
